# EUROPEAN PATENT SPECIFICATION

(11) **EP 4 099 910 B1**
(45) Date of publication and mention of the grant of the patent: **07.05.2025**
(21) Application number: 21703864.5
(22) Date of filing: 02.02.2021
(51) Int. Cl.: A61B 8/08, A61B 8/12, A61B 8/00, G06T 7/00, G16H 30/40, A61B 5/00

(54) **AUTOMATIC INTRALUMINAL IMAGING-BASED TARGET AND REFERENCE IMAGE FRAME DETECTION**
AUTOMATISCHE INTRALUMINALE ABBILDUNGSBASIERTE ZIEL- UND REFERENZBILDRAHMENERFASSUNG
DÉTECTION D'IMAGE DE RÉFÉRENCE ET DE CIBLE BASÉES SUR UNE IMAGERIE INTRALUMINALE AUTOMATIQUE

(30) Priority: 04.02.2020 US 202062969857 P
(43) Date of publication of application: 14.12.2022
(73) Proprietor: Koninklijke Philips N.V., 5656 AG Eindhoven (NL)
(72) Inventor: RAJGURU, Nikhil Sreedhar, 5656 AE Eindhoven (NL); NAIR, Anuja, 5656 AE Eindhoven (NL); COHEN, Asher, 5656 AE Eindhoven (NL); CHALYAN, David, 5656 AE Eindhoven (NL)
(74) Representative: Philips Intellectual Property & Standards
(86) International application number: PCT/EP2021/052344
(87) International publication number: WO 2021/156215

(56) References cited:
- WO-A2-2007/002685
- US-A1- 2014 276 085
- US-A1- 2016 157 798
- US-A1- 2019 282 211

## Description

### TECHNICAL FIELD

The present disclosure relates generally to intraluminal imaging data obtained by a catheter or guide wire positioned within a body lumen of patient. In particular, the present disclosure relates to automatic identification of target frames and reference frames of interest within an image data set based on the intraluminal imaging data obtained by the catheter or guide wire.

### BACKGROUND

Various types of intraluminal (also referred to as intravascular) imaging systems are used in diagnosing and treating diseases. For example, intravascular ultrasound (IVUS) imaging is widely used in interventional cardiology as a diagnostic tool for visualizing vessels within a body of a patient. This may aid in assessing diseased vessels, such as arteries and veins within the human body, to determine the need for treatment, to optimize treatment, and/or to assess the effectiveness of treatments.

In some cases, intraluminal imaging is carried out with an IVUS device including one or more ultrasound transducers. The IVUS device may be passed into the vessel and guided to the area to be imaged. The transducers emit ultrasonic energy and receive ultrasound echoes reflected from the vessel. The ultrasound echoes are processed to create an image of the vessel of interest.

Adoption of intraluminal imaging technology varies around the world and is underutilized in many parts of the world relative to the clinical evidence and benefits it provides. One barrier to the usage of intraluminal imaging is the manual selection of regions of interest. For example, some treatments (e.g., Percutaneous Coronary Intervention or PCI) may involve the placement of a stent in a vessel in order to widen the vessel in a location, and proper placement and dilation of the stent may be of major importance to favorable outcomes. IVUS-guided stent placement may be associated with more favorable outcomes than angiography-guided stent placement. However, correct placement of a stent involves manual selection of a target location (e.g., a target frame or a range of target frames of an IVUS pullback sequence) where lumen diameter or cross-sectional area is at a minimum, as well as proximal and distal reference locations (e.g., IVUS tomographic image frames proximal and distal to the target frame) where lumen diameter or cross-sectional area are within a healthy or expected range. In some instances, the stent is placed such that it fully covers the target location, and such that its edges coincide with the proximal and distal reference locations. However, there is no standardized protocol to select the targets of interest and potential references. This, along with barriers of image interpretation, are hindrances for novice and intermediate users while analyzing images acquired during an IVUS pullback sequence.

US 2019/282211 A1 discloses an angiographic image including a lumen and one or more intraluminal views of the lumen. The intraluminal views are transverse views of the lumen, which show different positions along the lumen. The lumen includes an area of interest that is identified by the system based on measured parameters of the lumen. A first intraluminal view correlates to a distal reference position with respect to the area of interest, a second intraluminal view correlates to a minimum lumen area of the area of interest, and a third intraluminal view correlates to a proximal reference position with respect to the area of interest. The intraluminal views are generated by the system after receiving imaging data from an intraluminal device being moved through the lumen. The intraluminal views are visually correlated to their positions along the angiographic view of the lumen, such as with dotted lines and matching colors

### SUMMARY

The invention is defined by the independent claims.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
**Figure 1** is a diagrammatic schematic view of an intraluminal imaging system incorporating the automatic frame identification system.
**Figure 2** illustrates a blood vessel including a stenosis.
**Figure 3** illustrates a blood vessel including a stenosis and propped open with a stent to re-establish a lumen within the blood vessel with the stenosis.
**Figure 4** is a screenshot from an example intraluminal imaging system incorporating a tomographic image of a lumen.
**Figure 5** shows an exemplary visualization showing a lumen with a stent.
**Figure 6** shows a flow diagram of an example automatic frame identification method.
**Figure 7** shows a flow diagram of an example automatic frame identification method.
**Figure 8** shows a flow diagram of target frame identification logic for an example automatic frame identification method.
**Figure 9** shows a flow diagram of proximal reference frame identification logic of an example automatic frame identification method.
**Figure 10** shows a flow diagram of distal reference frame identification logic of an example automatic frame identification method.
**Figure 11** shows a flow diagram of target merging logic for an example automatic frame identification method.
**Figure 12** shows a flow diagram of post-treatment logic for an example automatic frame identification method.
**Figure 13** is a schematic diagram of a processor circuit.

### DETAILED DESCRIPTION

Disclosed is an automatic frame identification system. At the present time, there is no standardized protocol to select the targets of interest and potential references. Attempts have been made to understand what the selection criteria may be, but no end-to-end protocol exists that covers all aspects of the selection process. This presents a hindrance for novice and intermediate users while analyzing images obtained during IVUS pullbacks. The present disclosure describes an end-to-end algorithm to take the measurements from each frame of an IVUS pullback and determine one or more targets of interest (e.g., stenoses) and respective proximal and distal reference (healthy) frames to help reduce the time taken in manual pullback analysis and to help guide the selection of landing zones for stents. In case of post-stent-placement procedures, a minimum stent area is indicated automatically. Various aspects of this algorithm can be configurable to match the individual's preferences and experience, to help a larger audience. The algorithm may also be referred to as a system, process, procedure, method, decision tree, or decision matrix.

The algorithm provides an automatic, standardized, quantitative method for finding one or more target frames of interest, and reference frames for each target, for a given pullback, starting from per-frame metrics. This process represents the secondary tier of steps to be followed after a full pullback image dataset has been acquired and all required per-frame metrics have been calculated for each frame in the data set, and may therefore sometimes be referred to as "secondary logic." This logic automatically identifies a target of interest in pre-treatment data (based for example on minimum lumen diameter or other considerations), a "minimum stent area" (MSA) frame in post-treatment data (based for example on minimum lumen diameter within the stent), and landing zones (e.g., start and end points for placement) for stents (based, for example, on being the closest tissue within the vessel that meets certain criteria to be considered healthy).

In addition to automatic detection of target and reference frames, the algorithm includes user settings to change the detection criteria. The algorithm can be applied to automatic pullback analysis and workflows, or any scenario where per-frame measurements are available for the whole pullback or captured image sequence, or portions thereof. The information derived by the algorithm may be used for example to determine the desired length and diameter of a stent, the desired location within the vessel to place the stent, and the desired degree of dilation of a stent after placement. The algorithm advises a clinician or other physician, and may serve as a starting point for clinical decision making.

The devices, systems, and methods described herein can include one or more features described in U.S. Provisional App. No. 62/643,105, filed 14 March 2018, U.S. Provisional App. No. 62/642,847, filed 14 March 2018, U.S. Provisional App. No. 62/712,009, filed 30 July 2018, and U.S. Provisional App. No. 62/643,366, filed 15 March 2018, and "Intravascular Ultrasound Versus Angiography-Guided Drug-Eluting Stent Implantation: The ULTIMATE Trial" (Junjie Zhang, et. al., JOURNAL OF THE AMERICAN COLLEGE OF CARDIOLOGY VOL. 72, NO. 24 , 2018, pp. 3126-3137).

The present disclosure aids substantially in repeatably identifying the best target and reference frames in an intraluminal image data set, by improving quantitative analysis and comparison of all frames in the data set. Implemented on an intraluminal imaging system in communication with an intraluminal imaging probe, the automatic frame identification system disclosed herein provides practical, quantitative guidance to clinicians in selecting dimensions and landing zones for stents and other treatments. This streamlined and augmented workflow transforms a tedious, error-prone manual process into a numerically rigorous automated selection, without the normally routine need to perform calculations or comparisons by hand, to flip through candidate images one by one, and to "eyeball" diseased and nearby healthy tissue to identify target and reference zones of interest. This unconventional approach improves the functioning of the intraluminal imaging system, by improving the speed and consistency of results associated with positive outcomes.

The automatic frame identification system may be implemented as a logic tree producing outputs viewable on a display, and operated by a control process executing on a processor that accepts user inputs from a keyboard, mouse, or touchscreen interface, and that is in communication with one or more intraluminal sensing devices. In that regard, the control process performs certain specific operations in response to different inputs, selections, or value edits made at different points in the execution. Certain structures, functions, and operations of the processor, display, sensors, and user input systems are known in the art, while others are recited herein to enable novel features or aspects of the present disclosure with particularity.

These descriptions are provided for exemplary purposes only, and should not be considered to limit the scope of the automatic frame identification system. Certain features may be added, removed, or modified without departing from the claimed subject matter

For the purposes of promoting an understanding of the principles of the present disclosure, reference will now be made to the embodiments illustrated in the drawings, and specific language will be used to describe the same. Any alterations and further modifications to the described devices, systems, and exemplary methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

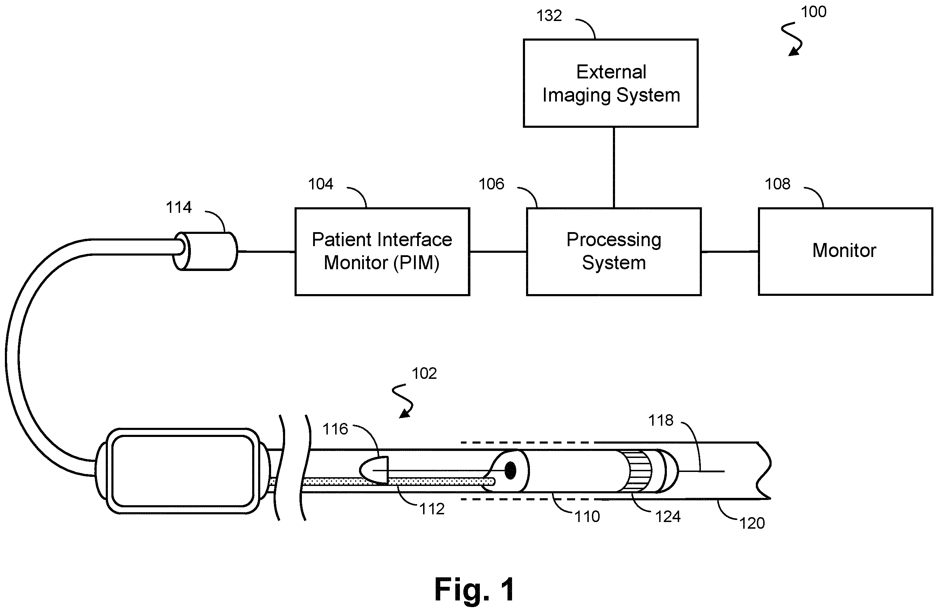

**Figure 1** is a diagrammatic schematic view of an intraluminal imaging system incorporating the automatic frame identification system, according to aspects of the present disclosure. The intraluminal imaging system 100 can be an intravascular ultrasound (IVUS) imaging system in some embodiments. The intraluminal imaging system 100 may include an intraluminal device 102, a patient interface module (PIM) 104, a console or processing system 106, a monitor 108, and an external imaging system 132 which may include angiography, ultrasound, X-ray, computed tomography (CT), magnetic resonance imaging (MRI), or other imaging technologies, equipment, and methods. The intraluminal device 102 is sized and shaped, and/or otherwise structurally arranged to be positioned within a body lumen of a patient. For example, the intraluminal device 102 can be a catheter, guide wire, guide catheter, pressure wire, and/or flow wire in various embodiments. In some circumstances, the system 100 may include additional elements and/or may be implemented without one or more of the elements illustrated in Figure 1. For example, the system 100 may omit the external imaging system 132.

The intraluminal imaging system 100 (or intravascular imaging system) can be any type of imaging system suitable for use in the lumens or vasculature of a patient. In some embodiments, the intraluminal imaging system 100 is an intraluminal ultrasound (IVUS) imaging system. In other embodiments, the intraluminal imaging system 100 may include for example systems configured for forward looking intravascular ultrasound (FL-IVUS) imaging, intravascular photoacoustic (IVPA) imaging, intracardiac echocardiography (ICE), transesophageal echocardiography (TEE), and/or other suitable imaging modalities.

It is understood that the system 100 and/or device 102 can be configured to obtain any suitable intraluminal imaging data. In some embodiments, the device 102 may include an imaging component of any suitable imaging modality, such as optical imaging, optical coherence tomography (OCT), etc. In some embodiments, the device 102 may include any suitable non-imaging component, including a pressure sensor, a flow sensor, a temperature sensor, an optical fiber, a reflector, a mirror, a prism, an ablation element, a radio frequency (RF) electrode, a conductor, or combinations thereof. Generally, the device 102 can include an imaging element to obtain intraluminal imaging data associated with the lumen 120. The device 102 may be sized and shaped (and/or configured) for insertion into a vessel or lumen 120 of the patient.

The system 100 may be deployed in a catheterization laboratory having a control room. The processing system 106 may be located in the control room. Optionally, the processing system 106 may be located elsewhere, such as in the catheterization laboratory itself. The catheterization laboratory may include a sterile field while its associated control room may or may not be sterile depending on the procedure to be performed and/or on the health care facility. The catheterization laboratory and control room may be used to perform any number of medical imaging procedures such as angiography, fluoroscopy, CT, IVUS, virtual histology (VH), forward looking IVUS (FL-IVUS), intraluminal photoacoustic (IVPA) imaging, a fractional flow reserve (FFR) determination, a coronary flow reserve (CFR) determination, optical coherence tomography (OCT), computed tomography, intracardiac echocardiography (ICE), forward-looking ICE (FLICE), intraluminal palpography, transesophageal ultrasound, fluoroscopy, and other medical imaging modalities, or combinations thereof. In some embodiments, device 102 may be controlled from a remote location such as the control room, such than an operator is not required to be in close proximity to the patient.

The intraluminal imaging device 102, PIM 104, monitor 108, and external imaging system 132 may be communicatively coupled directly or indirectly to the processing system 106. These elements may be communicatively coupled to the medical processing system 106 via a wired connection such as a standard copper link or a fiber optic link and/or via wireless connections using IEEE 802.11 Wi-Fi standards, Ultra Wide-Band (UWB) standards, wireless FireWire, wireless USB, or another high-speed wireless networking standard. The processing system 106 may be communicatively coupled to one or more data networks, e.g., a TCP/IP-based local area network (LAN). In other embodiments, different protocols may be utilized such as Synchronous Optical Networking (SONET). In some cases, the processing system 106 may be communicatively coupled to a wide area network (WAN). The processing system 106 may utilize network connectivity to access various resources. For example, the processing system 106 may communicate with a Digital Imaging and Communications in Medicine (DICOM) system, a Picture Archiving and Communication System (PACS), and/or a Hospital Information System via a network connection.

At a high level, an ultrasound intraluminal imaging device 102 emits ultrasonic energy from a transducer array 124 included in scanner assembly 110 mounted near a distal end of the intraluminal device 102. The ultrasonic energy is reflected by tissue structures in the medium (such as a lumen 120) surrounding the scanner assembly 110, and the ultrasound echo signals are received by the transducer array 124. The scanner assembly 110 generates electrical signal(s) representative of the ultrasound echoes. The scanner assembly 110 can include one or more single ultrasound transducers and/or a transducer array 124 in any suitable configuration, such as a planar array, a curved array, a circumferential array, an annular array, etc. For example, the scanner assembly 110 can be a one-dimensional array or a two-dimensional array in some instances. In some instances, the scanner assembly 110 can be a rotational ultrasound device. The active area of the scanner assembly 110 can include one or more transducer materials and/or one or more segments of ultrasound elements (e.g., one or more rows, one or more columns, and/or one or more orientations) that can be uniformly or independently controlled and activated. The active area of the scanner assembly 110 can be patterned or structured in various basic or complex geometries. The scanner assembly 110 can be disposed in a side-looking orientation (e.g., ultrasonic energy emitted perpendicular and/or orthogonal to the longitudinal axis of the intraluminal device 102) and/or a forward-looking looking orientation (e.g., ultrasonic energy emitted parallel to and/or along the longitudinal axis). In some instances, the scanner assembly 110 is structurally arranged to emit and/or receive ultrasonic energy at an oblique angle relative to the longitudinal axis, in a proximal or distal direction. In some embodiments, ultrasonic energy emission can be electronically steered by selective triggering of one or more transducer elements of the scanner assembly 110.

The ultrasound transducer(s) of the scanner assembly 110 can be a piezoelectric micromachined ultrasound transducer (PMUT), capacitive micromachined ultrasonic transducer (CMUT), single crystal, lead zirconate titanate (PZT), PZT composite, other suitable transducer type, and/or combinations thereof. In an embodiment the ultrasound transducer array 124 can include any suitable number of individual transducer elements or acoustic elements between 1 acoustic element and 1000 acoustic elements, including values such as 2 acoustic elements, 4 acoustic elements, 36 acoustic elements, 64 acoustic elements, 128 acoustic elements, 500 acoustic elements, 812 acoustic elements, and/or other values both larger and smaller.

The PIM 104 transfers the received echo signals to the processing system 106 where the ultrasound image (including the flow information) is reconstructed and displayed on the monitor 108. The console or processing system 106 can include a processor and a memory. The processing system 106 may be operable to facilitate the features of the intraluminal imaging system 100 described herein. For example, the processor can execute computer readable instructions stored on the non-transitory tangible computer readable medium.

The PIM 104 facilitates communication of signals between the processing system 106 and the scanner assembly 110 included in the intraluminal device 102. This communication may include providing commands to integrated circuit controller chip(s) within the intraluminal device 102, selecting particular element(s) on the transducer array 124 to be used for transmit and receive, providing the transmit trigger signals to the integrated circuit controller chip(s) to activate the transmitter circuitry to generate an electrical pulse to excite the selected transducer array element(s), and/or accepting amplified echo signals received from the selected transducer array element(s) via amplifiers included on the integrated circuit controller chip(s). In some embodiments, the PIM 104 performs preliminary processing of the echo data prior to relaying the data to the processing system 106. In examples of such embodiments, the PIM 104 performs amplification, filtering, and/or aggregating of the data. In an embodiment, the PIM 104 also supplies high- and low-voltage DC power to support operation of the intraluminal device 102 including circuitry within the scanner assembly 110.

The processing system 106 receives echo data from the scanner assembly 110 by way of the PIM 104 and processes the data to reconstruct an image of the tissue structures in the medium surrounding the scanner assembly 110. Generally, the device 102 can be utilized within any suitable anatomy and/or body lumen of the patient. The processing system 106 outputs image data such that an image of the vessel or lumen 120, such as a cross-sectional IVUS image of the lumen 120, is displayed on the monitor 108. Lumen 120 may represent fluid filled or fluid-surrounded structures, both natural and man-made. Lumen 120 may be within a body of a patient. Lumen 120 may be a blood vessel, such as an artery or a vein of a patient's vascular system, including cardiac vasculature, peripheral vasculature, neural vasculature, renal vasculature, and/or or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be used to examine man-made structures such as, but without limitation, heart valves, stents, shunts, filters and other devices.

The controller or processing system 106 may include a processing circuit having one or more processors in communication with memory and/or other suitable tangible computer readable storage media. The controller or processing system 106 may be configured to carry out one or more aspects of the present disclosure. In some embodiments, the processing system 106 and the monitor 108 are separate components. In other embodiments, the processing system 106 and the monitor 108 are integrated in a single component. For example, the system 100 can include a touch screen device, including a housing having a touch screen display and a processor. The system 100 can include any suitable input device, such as a touch sensitive pad or touch screen display, keyboard/mouse, joystick, button, etc., for a user to select options shown on the monitor 108. The processing system 106, the monitor 108, the input device, and/or combinations thereof can be referenced as a controller of the system 100. The controller can be in communication with the device 102, the PIM 104, the processing system 106, the monitor 108, the input device, and/or other components of the system 100.

In some embodiments, the intraluminal device 102 includes some features similar to traditional solid-state IVUS catheters, such as the EagleEye^{®} catheter available from Philips and those disclosed in U.S. Patent No. 7,846,101. For example, the intraluminal device 102 may include the scanner assembly 110 near a distal end of the intraluminal device 102 and a transmission line bundle 112 extending along the longitudinal body of the intraluminal device 102. The cable or transmission line bundle 112 can include a plurality of conductors, including one, two, three, four, five, six, seven, or more conductors.

The transmission line bundle 112 terminates in a PIM connector 114 at a proximal end of the intraluminal device 102. The PIM connector 114 electrically couples the transmission line bundle 112 to the PIM 104 and physically couples the intraluminal device 102 to the PIM 104. In an embodiment, the intraluminal device 102 further includes a guidewire exit port 116. Accordingly, in some instances the intraluminal device 102 is a rapid-exchange catheter. The guidewire exit port 116 allows a guidewire 118 to be inserted towards the distal end in order to direct the intraluminal device 102 through the lumen 120.

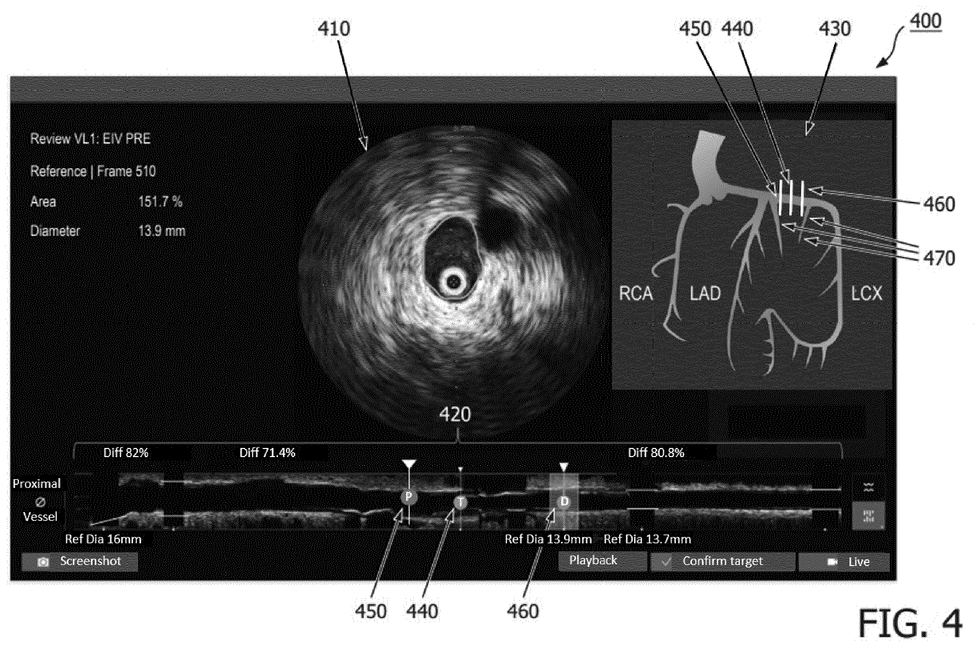

The monitor 108 may be a display device such as a computer monitor or other type of screen. The monitor 108 may be used to display selectable prompts, instructions, and visualizations of imaging data to a user. In some embodiments, the monitor 108 may be used to provide a procedure-specific workflow to a user to complete an intraluminal imaging procedure. This workflow may include performing a pre-stent plan to determine the state of a lumen and potential for a stent, as well as a post-stent inspection to determine the status of a stent that has been positioned in a lumen. The workflow may be presented to a user as any of the displays or visualizations shown in Figs. 4-5, or other displays.

The external imaging system 132 can be configured to obtain x-ray, radiographic, angiographic (e.g., with contrast), and/or fluoroscopic (e.g., without contrast) images of the body of a patient (including the vessel 120). External imaging system 132 may also be configured to obtain computed tomography images of the body of patient (including the vessel 120). The external imaging system 132 may include an external ultrasound probe configured to obtain ultrasound images of the body of the patient (including the vessel 120) while positioned outside the body. In some embodiments, the system 100 includes other imaging modality systems (e.g., MRI) to obtain images of the body of the patient (including the vessel 120). The processing system 106 can utilize the images of the body of the patient in conjunction with the intraluminal images obtained by the intraluminal device 102.

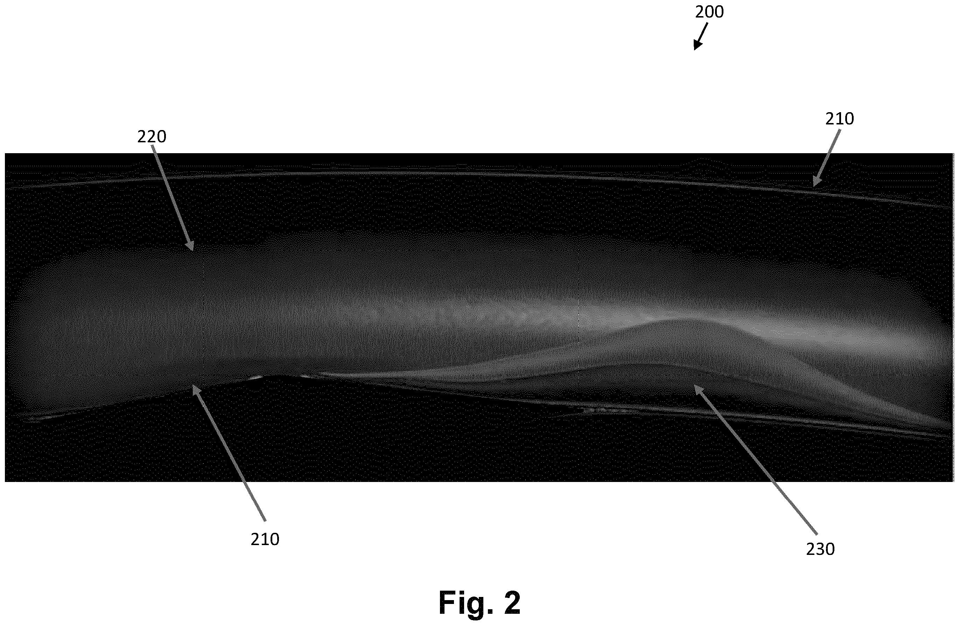

**Figure 2** illustrates a blood vessel 200 including a stenosis 230. The stenosis occurs between the vessel walls 210 and may restrict the flow of blood 220. Stenoses come in many types, including atherosclerosis.

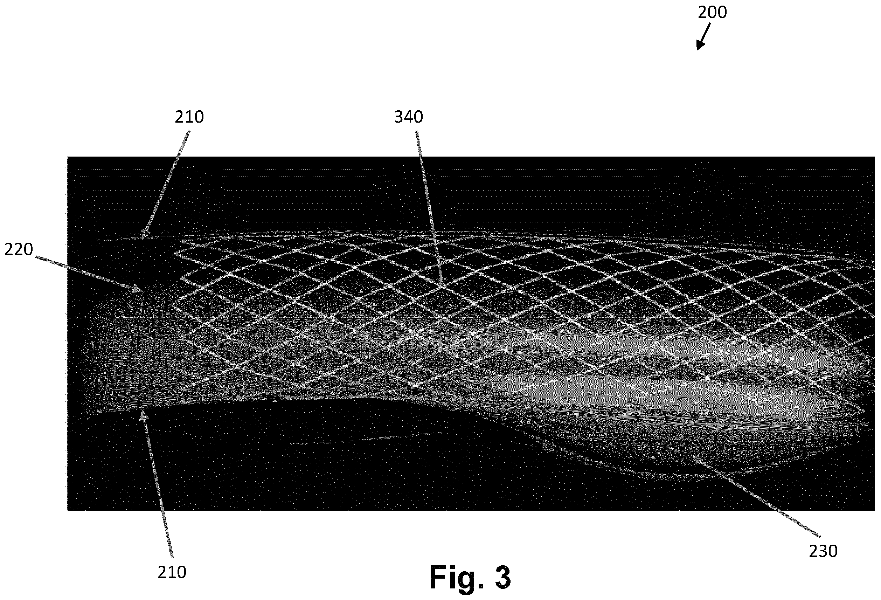

**Figure 3** illustrates a blood vessel 200 including a stenosis 230 and propped open with a stent 340 to re-establish a lumen within the blood vessel with the stenosis. The stent 340 compresses and arrests the stenosis 330, opening the blood vessel 300 and preventing the stenosis 230 from traveling through the blood vessel 200. The stent 340 also pushes the vessel walls 210 outward, thus reducing the flow restriction for the blood 220. Other treatment options for alleviating an occlusion may include but are not limited to thrombectomy, ablation, angioplasty, and pharmaceuticals. However, in a large majority of cases it may be highly desirable to obtain accurate and timely intravascular images of the affected area, along with accurate and detailed knowledge of the location of the affected area prior to, during, or after treatment. Inaccurate or imprecise location or orientation information for IVUS images may, for example, carry a risk of ablation or stenting of healthy tissue instead of diseased tissue during treatment.

**Figure 4** is a screenshot 400 from an example intraluminal imaging system 100 incorporating a tomographic image 410 of a lumen 120 in accordance with at least one embodiment of the present disclosure. Such tomographic images are radial or axial cross-sectional images, perpendicular to a longitudinal axis of the blood vessel, and the tomographic image frames can be generated, and measurements thereof can be automatically made, while the pullback is happening. The screenshot 400 also includes an angiogram image or graphical roadmap image 430, and an image longitudinal display (ILD) 420 composed of a plurality of stacked cross-sectional tomographic images from a pullback sequence, or graphical representations thereof. The ILD 420 is a longitudinal cross-sectional view, parallel to or including a longitudinal axis of the blood vessel. In some embodiments, the ILD 420 may comprise images from the pullback that are captured along the length of the vessel and shown along a length of the vessel in the longitudinal representation. In other embodiments, the ILD 420 may be a graphical representation of a geometrical property (e.g., diameter or cross-sectional area) of the vessel at different locations. Other information may be displayed instead of or in addition to that shown here.

Within the angiogram image or graphical roadmap image 430 are a target location marker 440, proximal reference location marker 450, distal reference location marker 460, and several vessel side branches 470. In some instances it may be undesirable for a target marker 440 or reference marker 450 or 460 to be co-located with a side branch 470, and thus in some embodiments the algorithm includes logic to avoid this. The ILD 420 also includes a target marker 440, proximal reference marker 450, and distal reference marker 460, each indicating a specific location or frame within the pullback sequence.

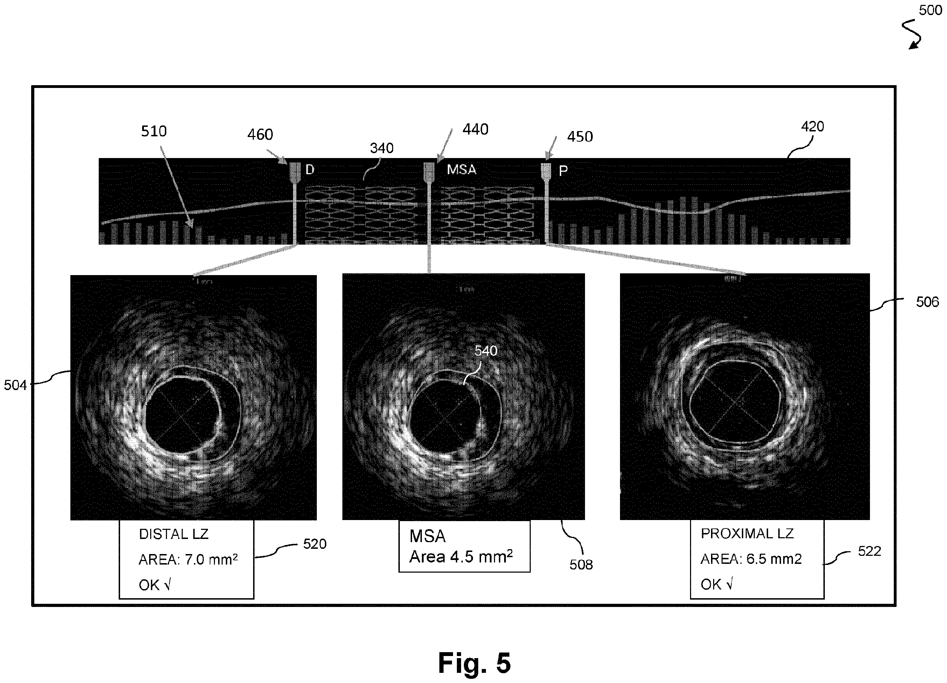

**Figure 5** shows an exemplary visualization 500 showing a lumen with a stent 340 according to aspects of the present disclosure. The visualization 500 includes a longitudinal representation or ILD 420, a distal reference frame 504, a target frame 508, and a proximal reference frame 506. Frames 504, 508, and 506 are tomographic images selected algorithmically from among the plurality of images in the IVUS pullback sequence. The ILD 420 includes a target marker 440, a proximal reference marker 450, and a distal reference marker 460. In a pre-treatment context, the target marker 440 and target frame 508 may represent an automatically detected location of minimum lumen diameter or area, whereas the proximal reference marker, distal reference marker, proximal reference frame, and distal reference frame may represent the automatically detected starting locations of healthy tissue and thus the desired locations for the proximal and distal edges of the stent. In a post-treatment context, the target marker 440 and target frame 508 may represent an automatically detected location of MSA, or minimum cross-sectional area of the stent 340, whereas the proximal reference marker 450, proximal reference frame 506, distal reference marker 460, and distal reference frame 504 may represent the automatically detected proximal and distal edges of the stent 340. In some instances, the region of the lumen 120 covered by the stent 340 is referred to as a landing zone or LZ.

The ILD 420, target marker 440, proximal reference marker 450, distal reference marker 460, distal reference frame 504, target frame 508, and proximal reference frame 506, displayed together on a single screen (or single user interface element), may collectively provide guidance to a clinician. In a pre-treatment context, this information may suggest to the clinician the desired dimensions (diameter and length) and landing zone of a stent 340 required to treat a stenosis 230. In a post-treatment context, this information may suggest to the clinician whether the stent 340 is properly positioned, whether the stent 340 requires dilation, and if so where, and by how much.

The imaging data (from which frames 504, 506, and 508 are automatically detected) may be collected by the device 102 as it is moved through the lumen 120, or through a stent 340 within the lumen 120. In some embodiments, the system may automatically detect the position of the stent 340 and display a visualization of the stent 340 on the visualization 500. Frames 504, 506, 508 may be visually correlated to the longitudinal view via the corresponding markers 460, 450, and 440. In other embodiments, frames 504, 506, 508 may be shown in the transverse with colors, symbols, shapes, text boxes, or other visual indicators.

The diameter and area of a stent 340 in each of the transverse views 504, 506, 508 may be automatically calculated and compared to other imaging data. For example, the calculated area and diameter of the each of the transverse views 504, 506, 508 may be compared to corresponding measurements in a pre-stent procedure. In this way, an operator may be able to check the effectiveness of the placed stent 340. Misalignment or malapposition of the stent 340 may also be automatically detected and displayed by the system. These aspects may be accompanied by visual cues such as highlighted areas or symbols and may have associated warnings to alert the operator of their presence.

In some embodiments, the ILD 420 includes a pressure graph 510 showing a measured pressure value for each location along the lumen 120 or vessel 200.

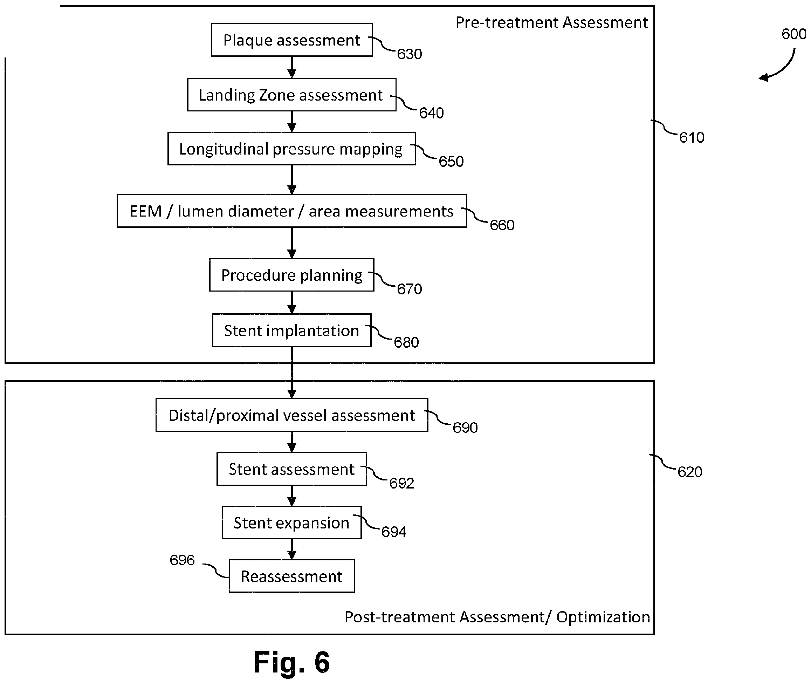

**Figure 6** shows a flow diagram 600 of an example automatic frame identification method. The flow diagram 600 includes pre-treatment assessment steps 610 (including steps 630-680) as well as post-treatment assessment and optimization steps 620 (including steps 690-696).

In step 630, the plaque burden and lumen area of the lumen 120 or vessel 200 are assessed for each frame of the image sequence (e.g., an IVUS pullback sequence).

In step 640, the collected image frames are analyzed to determine potential or desired landing zones for one or more stents 340.

In step 650, pressure readings along the length of the lumen 120 or vessel 200 are correlated with the positions of tomographic images taken during the pullback, and may for example be displayed as a longitudinal pressure graph 510 on an ILD 420.

In step 660, the cross-sectional areas and diameters of the vessel's external elastic membrane (EEM) and lumen regions are calculated for each frame of the pullback. Depending on the implementation, other anatomical features and measurements may also be calculated.

In step 670, the clinician plans the step-by-step details of a procedure to implant one or more stents, using visualizations, calculations, and frame selections provided by the automatic frame identification system.

In step 680, the clinician implants one or more stents in the vessel or lumen in the identified desired landing zone or zones, or uses a balloon to expand the vessel in the identified desired landing zone or zones.

In step 690, the lumen 120 or vessel 200 is assessed proximally and distally to the stent or stents, to ensure the tissue is healthy all the way up to the edges of the stent(s). "Dog boning", or widening of the ends of the stent vs. the center, may occur if the stent is misplaced or insufficiently dilated.

In step 692, the stent itself is assessed, to determine whether the minimum stent area (MSA) is within an expected or desired range. In many cases, it is desirable for the location of MSA to coincide with the location of a distal reference frame, since distal portions of a healthy vessel or lumen may be naturally narrower than proximal portions of the same vessel or lumen.

In step 694, if stent dilation is indicated, the clinician inserts a noncompliant balloon into the stent and inflates the balloon by a measured amount, thus dilating the stent to a desired diameter indicated by the automatic frame identification system.

In step 696, the stent and lumen are reassessed and, if any problems are detected, execution returns to step 690. Otherwise, the procedure ends.

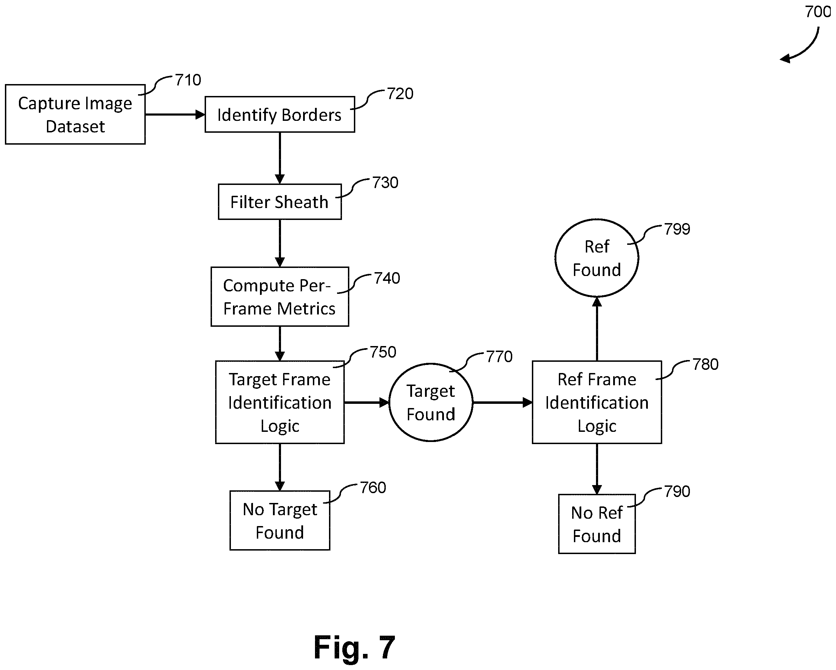

**Figure 7** shows a flow diagram 700 of an example automatic frame identification method.

In step 710, an image dataset is captured, for example during an IVUS pullback procedure that captures a sequence of tomographic images from within the lumen 120 or vessel 200 as the imaging probe 102 is pulled through it.

In step 720, the borders of the lumen 120 or vessel 200 are automatically identified in each frame of the sequence, using image recognition.

In step 730 frames that show the inside of a catheter sheath are automatically detected and deleted from the image sequence, so that they do not affect the operation of the algorithm. The sheath is a guide catheter that is inserted into the vessel, before the imaging catheter/guidewire. The imaging catheter/guidewire is guided through the sheath lumen to the location of the vessel. For example, the imaging element (ultrasound transducer, OCT element) is positioned distal of a lesion, while the distal end of the sheath is positioned proximal of the lesion. The imaging catheter/guide wire obtain imaging data during a pullback, such that the imaging element is moved longitudinally, proximally towards the sheath. This is why the proximal side of the pullback sequence could include tomographic images representative of (e.g., captured within) the sheath. In a pullback, the image frames with the sheath are at the end. If the user was moving the imaging catheter/guide from proximal to distal direction (opposite direction of pullback), the image frames with the sheath would be at the beginning. In some instances, the imaging probe is extended from the catheter sheath to a certain point within the lumen or vessel, and then pulled back through the lumen or vessel until it re-enters the catheter sheath. Thus, sheath frames may be found near the beginning or end of an image sequence. It is noted that in some embodiments, step 730 can occur before step 720.

In step 740 per-frame metrics are computed for each non-deleted frame in the sequence. Per-frame metrics may include plaque burden (PB) and lumen area (LA), and other anatomical measurements.

In step 750, a target frame is automatically identified based on the per-frame metrics, as described below. The target frame represents an anatomical region of interest such as a stenosis. In an example, this region of interest is a potential or recommended treatment location, such as a location corresponding to an image frame with a relatively smaller measurement of diameter, area or a relatively larger plaque burden, etc. If a target is found, execution moves to step 770. If not, execution moves to step 760.

In step 760, the method reports that it has been unable to identify a target frame, based on the specified criteria as described below. This may, for example, encourage a clinician to adjust the search criteria so that a target frame can be identified. Alternatively, it may imply that the disease state of the vessel or lumen is insufficient to warrant treatment.

In step 770, the algorithm reports the identified target frame, including relevant per-frame metrics as described above.

In step 780, the proximal and distal reference frames, representing the nearest healthy tissue to the target frame, are automatically identified, as described below. If one or both references are not found, execution moves to step 790. Otherwise, execution moves to step 799.

In step 790, the method reports that it has been unable to identify the required reference frames, based on the specified criteria as described below. This may, for example, encourage a clinician to adjust the search criteria so that the proximal and distal frames can be identified.

In step 799, the method reports (e.g., displays) the identified proximal and distal reference frames, along with the relevant per-frame metrics.

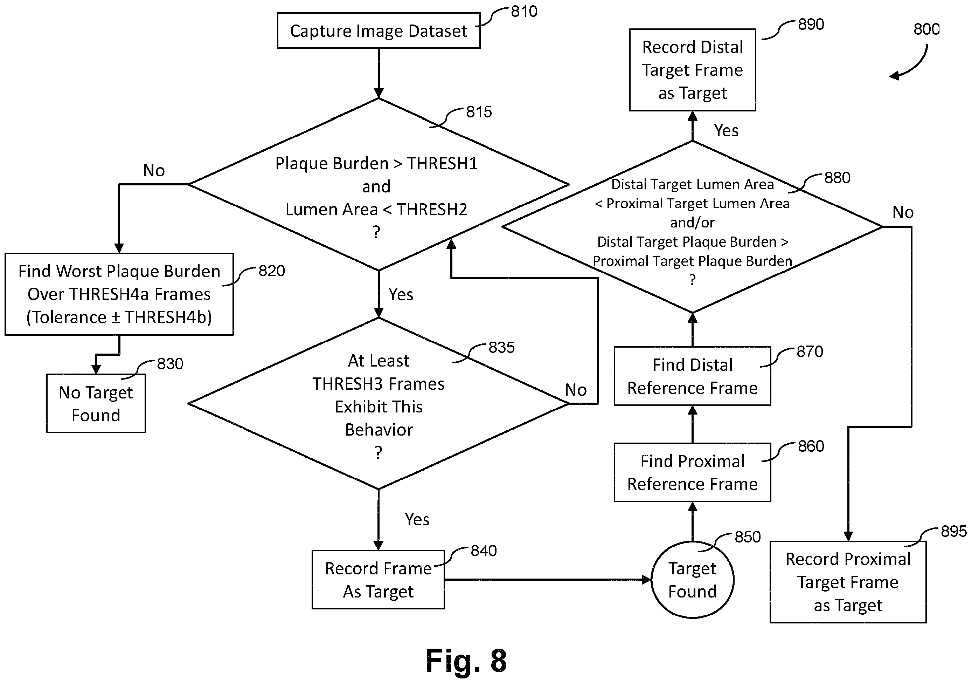

**Figure 8** shows a flow diagram 800 of target frame identification logic for an example automatic frame identification method.

In step 810, the system captures an image dataset as described above.

In step 815, the system examines each frame of the dataset until it finds a frame with a plaque burden (PB) greater than a first threshold THRESH1 and a lumen area (LA) less than a second threshold THRESH2. In an example, THRESH1 and THRESH2 are user-editable parameters, although the system may also define a default value for each (e.g., THRESH1 = 70% and THRESH2 = 4 mm² for exemplary coronary vasculature, although other values may be used depending on the anatomy being considered). If no such candidate frame can be identified in the dataset, execution passes to step 820. If such a candidate frame is identified, execution passes to step 835.

In step 820, the algorithm has failed to identify a target frame that meets the specified criteria, so the algorithm instead finds a sequence of THRESH4a frames with the largest plaque burden (with a tolerance of ± THRESH4b) in the image dataset, and reports this information to the clinician. This information may be used for example in determining whether to revise the target frame detection criteria, or whether to postpone treatment. In an example, THRESH4a and THRESH4b are user-editable parameters, although the system may also specify default values.

In step 830 the algorithm reports that no target frame has been found.

In step 835, once a potential target frame has been identified in step 815, the algorithm checks the next THRESH3 frames to see if they also meet the criteria for a target frame. If this is not the case, then execution returns to step 815 to continue scanning frames. If it is the case, then execution proceeds to step 840. In an example, THRESH3 is a user-editable parameter, although the system may also specify a default value.

In step 840, the identified frame is recorded as a target frame, and execution proceeds to step 850.

In step 850, the system reports the identified target frame, along with its relevant per-frame metrics (e.g., plaque burden and lumen area).

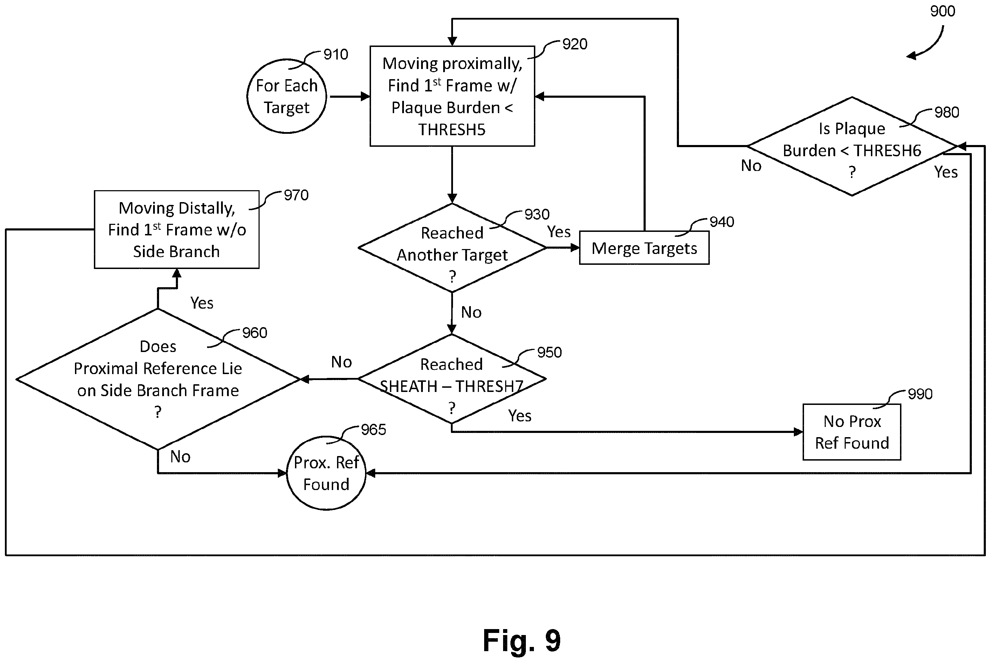

In step 860, the system executes proximal reference frame identification logic as described below in Figure 9.

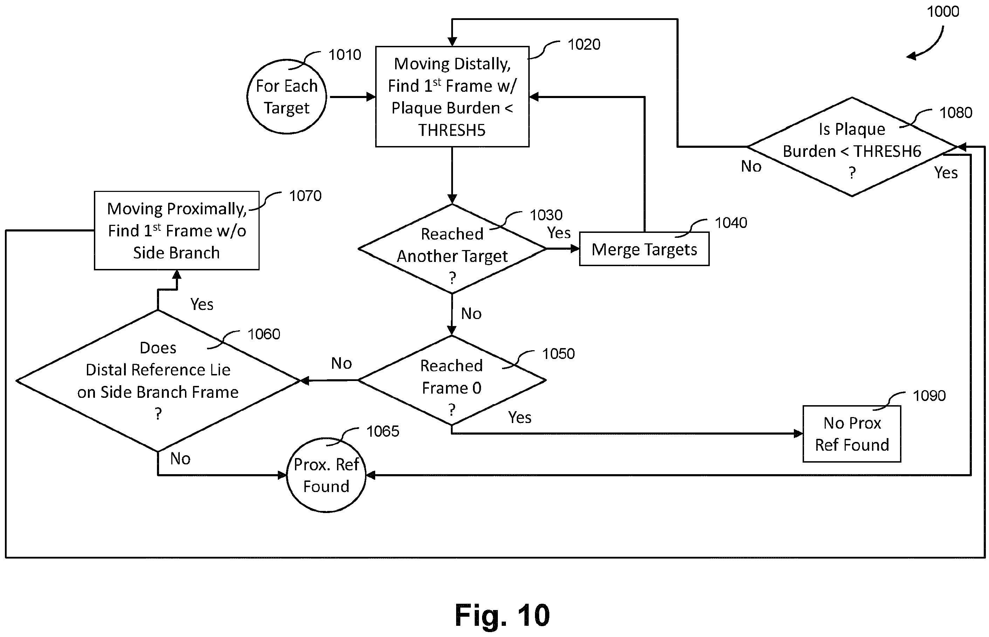

In step 870, the system executes distal reference frame identification logic as described below in Figure 10. It is noted that in some embodiments, step 870 may occur before step 860. It is further noted that the per-frame metrics of either the proximal reference frame or the distal reference frame may be compared with the target frame to compute values such as percent stenosis.

In step 880, the method determines which target frame (distal or proximal) will be in case no reference is found between two targets (e.g., a condition where two targets are to be merged). If the distal target frame shows at least one of a smaller lumen area or a greater plaque burden than the proximal target frame, then the distal frame is recorded as the absolute target frame in step 890. Otherwise, the proximal frame is recorded as the absolute target frame in step 895.

It is noted that the method may also be configured such that the terms "distal" and "proximal" are swapped in at least steps 880, 890, and 895.

**Figure 9** shows a flow diagram 900 of proximal reference frame identification logic of an example automatic frame identification method.

In step 910, the method initiates the proximal reference frame identification logic for each target identified in the dataset.

In step 920, the method identifies the first frame that is proximal of the target and that has a plaque burden less than THRESH5. In an example, THRESH5 is a user-editable parameter, although the system may also define a default value (e.g., 40%).

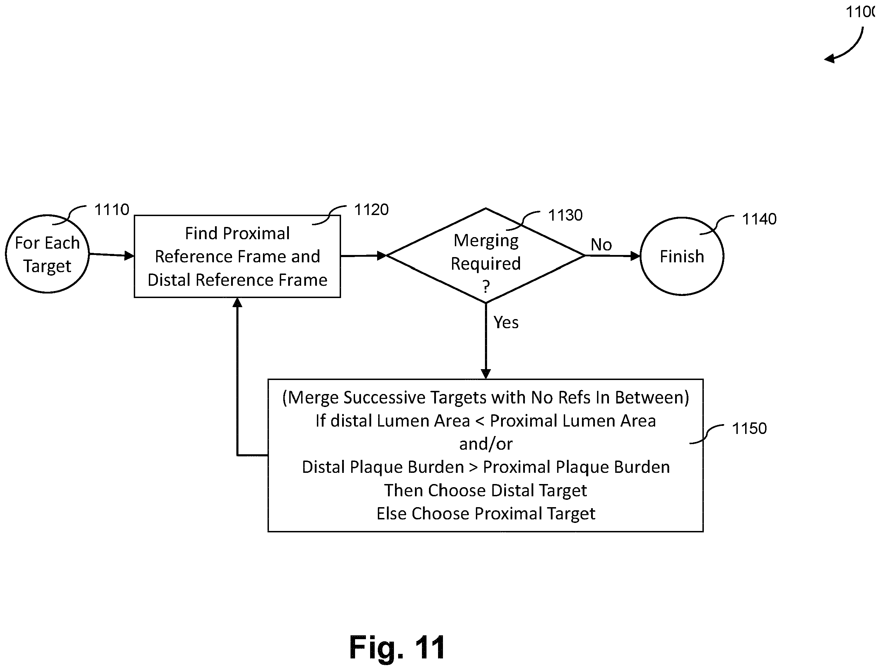

In step 930, the method determines whether another frame meeting the target criteria of Figure 8 can be found before encountering a frame that satisfies the THRESH5 criterion. If so, then execution moves to step 940, where this potential target frame is merged with the existing target as described below in Figure 11, and execution then returns to step 920. If an intervening target frame is not found, execution proceeds to step 950.

In step 950, the method checks to see whether the frame-by-frame search has reached within THRFSH0 frames of the start of the sheath. If yes, execution proceeds to step 990, and the method reports that no proximal reference was found. If no, then the identified candidate proximal reference is referred to step 960. In an example, THRESH0 is a user editable parameter, although the system may also specify a default value.

In step 960, the method checks to see whether the identified candidate proximal reference frame lies on a side branch 470 of the lumen 120 or vessel 200 (as shown for example in Fig 4). If yes, execution proceeds to step 970. If no, execution proceeds to step 965, wherein the identified candidate proximal reference is marked as the proximal reference frame.

In step 970, the method searches back (e.g., distally) to find the closest frame that does not include a side branch 470, which then becomes the candidate proximal reference frame.

In step 980, the method determines whether the candidate proximal reference frame shows a plaque burden less than THRESH6. If no, then execution returns to step 920. If yes, then execution proceeds to step 965, wherein the identified candidate proximal reference is marked as the proximal reference frame. In an example, THRESH6 is a user-editable parameter, although the method may also specify a default value. In other embodiments, depending on the implementation, the proximal reference frame may represent the nearest healthy tissue proximal of the target frame, where "healthy" is defined either as completely healthy tissue, or as tissue whose disease burden is less than that of the target frame and satisfies a criterion associated with at least one of a plaque burden or a lumen cross-sectional area.

**Figure 10** shows a flow diagram 1000 of distal reference frame identification logic of an example automatic frame identification method.

In step 1010, the method initiates the distal reference frame identification logic for each target identified in the dataset.

In step 1020, the method identifies the first frame that is distal to the target and that has a plaque burden less than THRESH5. In an example, THRESH5 is a user-editable parameter, although the method may also define a default value.

In step 1030, the method determines whether another frame meeting the target criteria of Figure 8 can be found before encountering a frame that satisfies the THRESH5 criterion. If so, then execution moves to step 1040, where this potential target frame is merged with the existing target as described below in Figure 11, and execution then returns to step 1020. If an intervening target frame is not found, execution proceeds to step 1050.

In step 1050, the method checks to see whether the frame-by-frame search has reached the end of the image sequence (e.g., the first or last frame in the sequence). If yes, execution proceeds to step 1090, and the method reports that no distal reference was found. If no, then the identified candidate distal reference is referred to step 1060.

In step 1060, the method checks to see whether the identified candidate distal reference frame lies on a side branch 470 of the lumen 120 or vessel 200 (as shown for example in Fig 4). If yes, execution proceeds to step 1070. If no, execution proceeds to step 1065, wherein the identified candidate distal reference is marked as the distal reference frame.

In step 1070, the method searches back (e.g., proximally) to find the closest frame that does not include a side branch 470, which then becomes the candidate distal reference frame.

In step 1080, the method determines whether the candidate distal reference frame shows a plaque burden less than THRESH6. If no, then execution returns to step 1020. If yes, then execution proceeds to step 1065, wherein the identified candidate distal reference frame is marked as the distal reference frame. In an example, THRESH6 is a user-editable parameter, although the method may also specify a default value. In other embodiments, depending on the implementation, the distal reference frame may represent the nearest healthy tissue distal of the target frame, where "healthy" is defined either as completely healthy tissue, or as tissue whose disease burden is less than that of the target frame and satisfies a criterion associated with at least one of a plaque burden or a lumen cross-sectional area.

**Figure 11** shows a flow diagram 1100 of target merging logic for an example automatic frame identification method.

In step 1110, the method initiates the steps for each target identified in the dataset.

In step 1120, the proximal and distal reference frames are identified, as shown above in Figures 9 and 10.

In step 1130, the method determines whether the merging of target frames is required (as shown above, for example in steps 930 and 1030). If no, execution proceeds to step 1140 (finish), and the target merging logic takes no action for the current target. If yes, execution proceeds to step 1150.

In step 1150, successive targets with no proximal or distal reference in between them are "merged", or filtered to select one target frame over the other using the merge criteria. The targets are filtered two at a time; in each comparison there is a more proximal target and a more distal target. If the distal target frame has at least one of a smaller lumen area or a larger plaque burden than the proximal target frame, then the distal target frame is selected as the absolute target frame of that region to be displayed, and the per-frame values of the distal target are used as the target per-frame values; otherwise, the proximal target frame is selected as the absolute target frame of that region to be displayed, and the per-frame values of the proximal frame are used as the target per-frame values. This "merging" or elimination continues until all the candidate target frames without reference frames between them are filtered. The target merging logic is then complete. In some embodiments, the target is selected based on both the greatest plaque burden and the smallest lumen area. In some embodiments, the greatest plaque burden is weighted more heavily than the smallest lumen area. In other embodiments, the smallest lumen area is weighted more heavily than the greatest plaque burden. In still other embodiments, the selected lumen satisfies another criterion associated with at least one of a plaque burden or a lumen cross-sectional area.

It is noted that the method may also be configured such that the terms "distal" and "proximal" are swapped in at least step 1150.

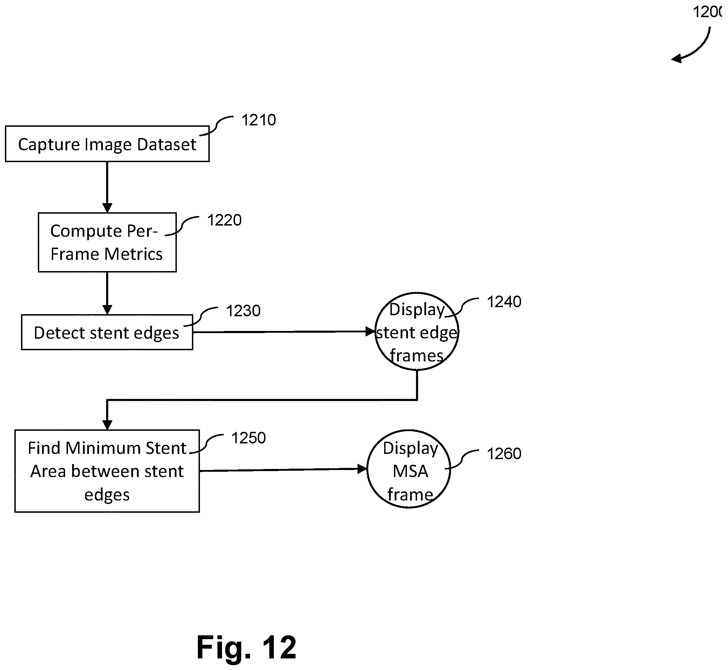

**Figure 12** shows a flow diagram 1200 of post-treatment logic for an example automatic frame identification method.

In step 1210, a post-treatment image dataset is captured as described above.

In step 1220, the per-frame metrics are computer as described above.

In step 1230, stents (if any) are identified using image recognition, and image frames containing the proximal and distal stent edges are marked as proximal and distal reference frames.

In step 1240, the proximal and distal reference frames containing the stent edges are displayed (as shown for example in Figure 5).

In step 1250, the minimum stent area (MSA) frame is identified based on the per-frame metrics, and marked as a target frame.

In step 1260, the target frame is displayed (as shown for example in Figure 5).

It is noted that the MSA may be detected and displayed within the identified stent region and could also be detected and displayed from one or more sub-regions within the stent. The sub-region or sub-regions could be automatically identified or could be defined by user-inputs based on criteria or thresholds that could be edited by a user. For example, the sub-regions can be a proximal region, a central region, and/or a distal region of the stent. The sub-regions can be identified based on a length of the stent in some embodiments using, for example, intraluminal imaging data, extraluminal imaging data, and/or a length of the stent provided to the processor circuit. Detecting and displaying the MSA within the sub-region(s) within the stent can be used to verify that different portions of the stent have been properly expanded within the blood vessel.

One or more of methods 600, 700, 800, 900, 1000, 1100, and/or 1200 can include a step of comparing a measurement and/or anatomical quantity to a threshold. In some embodiments, such a step or other step can include determining a value (e.g., a numerical value) of the measurement and a value (e.g., a numerical value) of the threshold. The comparison can be determining when the value of the measurement reaches the value of the threshold (e.g., when the measurement equals the threshold, when the measurement exceeds the threshold, and/or when the measurement is less than the threshold).

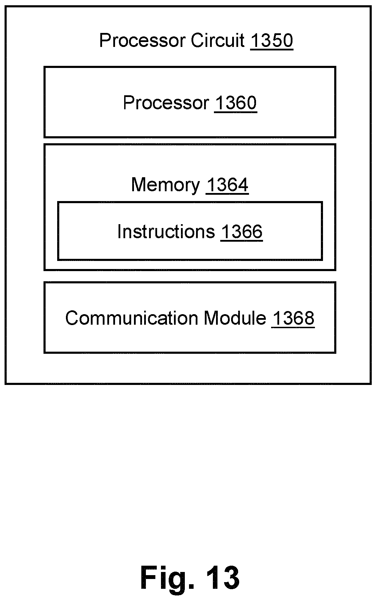

**Figure 13** is a schematic diagram of a processor circuit 1350. The processor circuit receives imaging data from the intraluminal device. The processor circuit 1350 may be implemented in the ultrasound imaging system 100, or other devices or workstations (e.g., third-party workstations, network routers, etc.) as necessary to implement the method. As shown, the processor circuit 1350 may include a processor 1360, a memory 1364, and a communication module 968. These elements may be in direct or indirect communication with each other, for example via one or more buses.

The processor 1360 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1360 may also comprise another hardware device, a firmware device, or any combination thereof configured to perform the operations described herein. The processor 1360 may also be implemented as a combination of computing devices, e.g., a combination of a DSP and a microprocessor, a plurality of microprocessors, one or more microprocessors in conjunction with a DSP core, or any other such configuration.

The memory 1364 may include a cache memory (e.g., a cache memory of the processor 1360), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1364 includes a non-transitory computer-readable medium. The memory 1364 may store instructions 1366. The instructions 1366 may include instructions that, when executed by the processor 1360, cause the processor 1360 to perform the operations described herein. Instructions 1366 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1368 can include any electronic circuitry and/or logic circuitry to facilitate direct or indirect communication of data between the processor circuit 1350, and other processors or devices. In that regard, the communication module 1368 can be an input/output (I/O) device. In some instances, the communication module 1368 facilitates direct or indirect communication between various elements of the processor circuit 1350 and/or the ultrasound imaging system 100. The communication module 1368 may communicate within the processor circuit 1350 through numerous methods or protocols. Serial communication protocols may include but are not limited to US SPI, I²C, RS-232, RS-485, CAN, Ethernet, ARINC 429, MODBUS, MIL-STD-1553, or any other suitable method or protocol. Parallel protocols include but are not limited to ISA, ATA, SCSI, PCI, IEEE-488, IEEE-1284, and other suitable protocols. Where appropriate, serial and parallel communications may be bridged by a UART, USART, or other appropriate subsystem.

External communication (including but not limited to software updates, firmware updates, or readings from the ultrasound device) may be accomplished using any suitable wireless or wired communication technology, such as a cable interface such as a USB, micro USB, Lightning, or FireWire interface, Bluetooth, Wi-Fi, ZigBee, Li-Fi, or cellular data connections such as 2G/GSM, 3G/UMTS, 4G/LTE/WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

In some implementations, the logic branches may be different than shown herein. Additional steps may occur, and some steps listed herein may not be performed. It should further be understood that the described technology may be employed in numerous types of intraluminal procedures that require intraluminal imaging. Accordingly, the logical operations making up the embodiments of the technology described herein are referred to variously as operations, steps, objects, elements, components, or modules. Furthermore, it should be understood that these may occur or be performed in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the automatic frame identification system. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." Unless otherwise noted in the claims, stated values shall be interpreted as illustrative only and shall not be taken to be limiting.

The above specification, examples and data provide a complete description of the structure and use of exemplary embodiments of the automatic frame identification system as defined in the claims. Various embodiments of the claimed subject matter have been described above with a certain degree of particularity, or with reference to one or more individual embodiments.

## Claims

1. An automatic frame identification system, comprising a processor circuit (106) configured to:
identify as a target frame (508) representative of a region of interest, an image frame of a plurality of image frames generated using imaging data obtained by an intraluminal imaging device (102) while positioned within a lumen, wherein the image frame satisfies a first criterion associated with an anatomical feature, wherein the identification is based on a comparison to a threshold of an automatic measurement of the anatomical feature in the plurality of image frames; and
perform at least one of:
- identifying as a candidate proximal reference frame (506) representative of a nearest tissue of the lumen proximal to the target frame, an image frame that satisfies a second criterion associated with the anatomical feature;
- if the candidate proximal reference frame lies on a side branch, search distally to find the closest image frame that does not include the side branch and assign the closest image frame as the proximal reference frame;
- if the candidate proximal reference frame does not lie on a side branch, assign the candidate proximal reference frame as the proximal reference frame and
- identifying as a candidate distal reference frame (504) representative of a nearest tissue of the lumen distal to the target frame, an image frame that satisfies a third criterion associated with the anatomical feature;
- if the candidate distal reference frame lies on a side branch, search proximally to find the closest image frame that does not include the side branch and assign the closest image frame as the distal reference frame; and
- if the candidate distal reference frame does not comprise a side branch, assign the candidate distal reference frame as the distal reference frame; and output, to a display in communication with the processor circuit, a single screen display including:
- the target frame, and any assigned proximal reference frame and any assigned distal reference frame; and
- a graphical representation (420) of the lumen showing a respective position of the target frame and any assigned proximal reference frame and any assigned distal reference frame.

2. The automatic frame identification system of claim 1, wherein the processor circuit is further configured to:
Perform at least one of:
- before assigning the closest image frame as the proximal reference frame or as the distal reference frame, identify whether the closest image frame satisfies a further criterion associated with the anatomical feature; and
- if the closest image frame satisfies the criterion, assign the closest reference frame as the proximal reference frame or the distal reference frame.

3. The automatic frame identification system of claim 1 or 2, wherein the anatomical feature consists of at least one chosen from the group consisting of: a plaque burden and a cross-sectional area of the lumen.

4. The automatic frame identification system of any one of claims 1 to 3, wherein the processor circuit is further configured to:
generate the plurality of image frames using the imaging data;
automatically measure the anatomical feature in the plurality of image frames.
communicate with an intraluminal imaging device configured to obtain imaging data associated with a lumen of a patient while positioned within the lumen

5. The automatic frame identification system of any one of the previous claims, wherein the automatic measurement is in response to generating the plurality of image frames.

6. The automatic frame identification system of claim 1, comprising the display in communication with the processor circuit.

7. An intraluminal imaging system comprising:
an automatic frame identification system of any one of claims 1 to 6; and
the intraluminal imaging device.

8. A method for automatic frame identification, comprising, using a processor circuit (106):
identifying as a target frame (508) representative of a region of interest, an image frame of a plurality of image frames generated using imaging data obtained by an intraluminal imaging device (102) while positioned within a lumen, wherein the image frame satisfies a first criterion associated with an anatomical feature, wherein the identification is based on a comparison to a threshold of an automatic measurement of the anatomical feature in the plurality of image frames; and
performing at least one of:
- identifying as a candidate proximal reference frame (506) representative of a nearest tissue of the lumen proximal to the target frame, an image frame of the plurality of image frames that satisfies a second criterion associated with the anatomical feature;
- if the candidate proximal reference frame lies on a side branch, searching distally to find the closest image frame that does not include the side branch and assigning the closest image frame as the proximal reference frame;
- if the candidate proximal reference frame does not lie on a side branch, assigning the candidate proximal reference frame as the proximal reference frame
and
- identifying as a candidate distal reference frame (504) representative of a nearest tissue of the lumen distal to the target frame, an image frame of the plurality of image frames that satisfies a third criterion associated with the anatomical feature;
- if the candidate distal reference frame lies on a side branch, searching proximally to find the closest image frame that does not include the side branch and assigning the closest image frame as the distal reference frame; and
- if the candidate distal reference frame does not comprise a side branch, assigning the candidate distal reference frame as the distal reference frame; and outputting, to a display in communication with the processor circuit, a single screen display including:
the target frame, and any assigned proximal reference frame and any assigned distal reference frame; and
a graphical representation of the lumen (420) showing a respective position of the target frame and any assigned proximal reference frame and any assigned distal reference frame.

9. The method of claim 8, further comprising, using the processor circuit:
Performing at least one of:
- before assigning the closest image frame as the proximal reference frame or as the distal reference frame, identifying whether the closest image frame satisfies a further criterion associated with the anatomical feature; and
- if the closest image frame satisfies the criterion, assigning the closest reference frame as the proximal reference frame or the distal reference frame.

10. The method of claim 8 or 9, wherein the anatomical feature consists of at least one chosen from the group consisting of a plaque burden and cross-sectional area of the lumen.

11. The method of any one of claims 8 to 10, wherein the method further comprises:
generating, using the processor circuit in communication with the intraluminal imaging device, the plurality of image frames using the imaging data;
automatically measuring, using the processor circuit, the anatomical feature in the plurality of image frames.

12. The method of any one of the claims 8 to 11, wherein the automatic measurement is in response to generating the plurality of image frames.

13. The method of any one of the claims 8 to 12, wherein the single screen display is displayed to a user using the display.

14. A computer program comprising instructions that, when executed by a computer system or apparatus, causes the computer system or apparatus to perform any one of the methods of claims 8 to 13.

## Patentansprüche

1. Automatisches Frame-Identifikationssystem, das eine Prozessorschaltung (106) umfasst, die für Folgendes konfiguriert ist:
Identifizieren, als ein Zielframe (508), das einen Bereich von Interesse darstellt, eines Bildframes aus einer Vielzahl von Bildframes, die unter Verwendung von Bildgebungsdaten erzeugt wurden, die von einer intraluminalen Bildgebungsvorrichtung (102) erhalten wurden, während sie in einem Lumen positioniert war, wobei das Bildframe ein erstes Kriterium erfüllt, das einem anatomischen Merkmal zugeordnet ist, wobei die Identifizierung auf einem Vergleich mit einem Schwellenwert einer automatischen Messung des anatomischen Merkmals in der Vielzahl von Bildframes basiert; und
Durchführen mindestens eines des Folgenden:
- Identifizieren eines Bildframes, das ein zweites Kriterium erfüllt, das dem anatomischen Merkmal zugeordnet ist, als ein proximales Kandidatenreferenzframe (506), das ein nächstgelegenes Gewebe des Lumens proximal zu dem Zielframe darstellt;
- wenn das proximale Kandidatenreferenzframe auf einem Seitenast liegt, distales Suchen, um das nächstgelegene Bildframe zu finden, das den Seitenast nicht beinhaltet, und Zuweisen des nächstgelegenen Bildframes als das proximale Referenzframe;
- wenn das proximale Kandidatenreferenzframe nicht auf einem Seitenast liegt, Zuweisen des proximalen Kandidatenreferenzframes als das proximale Referenzframe
und
- Identifizieren eines Bildframes, das ein drittes Kriterium erfüllt, das dem anatomischen Merkmal zugeordnet ist, als ein distales Kandidatenreferenzframe (504), das ein nächstgelegenes Gewebe des Lumens distal zu dem Zielframe darstellt;
- wenn das distale Kandidatenreferenzframe auf einem Seitenast liegt, proximales Suchen, um das nächstgelegene Bildframe zu finden, das den Seitenast nicht beinhaltet, und Zuweisen des nächstgelegenen Bildframes als das distale Referenzframe; und
- wenn das distale Kandidatenreferenzframe keinen Seitenast umfasst, Zuweisen des distalen Kandidatenreferenzframes als das distale Referenzframe; und
Ausgeben, an eine Anzeige in Kommunikation mit der Prozessorschaltung, einer Einzelbildschirmanzeige, die Folgendes beinhaltet:
- das Zielframe und jegliches zugewiesenes proximales Referenzframe und jegliches zugewiesenes distales Referenzframe; und
- eine grafische Darstellung (420) des Lumens, die eine jeweilige Position des Zielframes und jeglichen zugewiesenen proximalen Referenzframes und jeglichen zugewiesenen distalen Referenzframes zeigt.

2. Automatisches Frame-Identifikationssystem nach Anspruch 1, wobei die Prozessorschaltung weiter für Folgendes konfiguriert ist:
Durchführen mindestens eines des Folgenden:
- vor dem Zuweisen des nächstgelegenen Bildframes als das proximale Referenzframe oder das distale Referenzframe, Identifizieren, ob das nächstgelegene Bild ein weiteres Kriterium erfüllt, das dem anatomischen Merkmal zugeordnet ist; und
- wenn das nächstgelegene Bildframe das Kriterium erfüllt, Zuweisen des nächstgelegenen Referenzframes als das proximale Referenzframe oder das distale Referenzframe.

3. Automatisches Frame-Identifikationssystem nach Anspruch 1 oder 2, wobei das anatomische Merkmal aus mindestens einem Merkmal besteht, das aus der Gruppe ausgewählt wird, die aus Folgendem besteht: einer Plaque-Belastung und einer Querschnittsfläche des Lumens.

4. Automatisches Frame-Identifikationssystem nach einem der Ansprüche 1 bis 3, wobei die
Prozessorschaltung weiter für Folgendes konfiguriert ist:
Erzeugen der Vielzahl von Bildframes unter Verwendung der Bildgebungsdaten;
automatisches Messen des anatomischen Merkmals in der Vielzahl von Bildframes;
Kommunizieren mit einer intraluminalen Bildgebungsvorrichtung, die dazu konfiguriert ist, Bildgebungsdaten zu erhalten, die einem Lumen eines Patienten zugeordnet sind, während diese im Lumen positioniert ist.

5. Automatisches Frame-Identifikationssystem nach einem der vorstehenden Ansprüche, wobei die automatische Messung als Reaktion darauf erfolgt, dass die Vielzahl von Bildframes erzeugt wird.

6. Automatisches Frame-Identifikationssystem nach Anspruch 1, das die Anzeige in Kommunikation mit der Prozessorschaltung umfasst.

7. Intraluminales Bildgebungssystem, das Folgendes umfasst:
ein automatisches Frame-Identifikationssystem nach einem der Ansprüche 1 bis 6; und
die intraluminale Bildgebungsvorrichtung.

8. Verfahren zur automatischen Frame-Identifikation, das unter Verwendung einer Prozessorschaltung (106) Folgendes umfasst:
Identifizieren, als ein Zielframe (508), das einen Bereich von Interesse darstellt, eines Bildframes aus einer Vielzahl von Bildframes, die unter Verwendung von Bildgebungsdaten erzeugt wurden, die von einer intraluminalen Bildgebungsvorrichtung (102) erhalten wurden,
während sie in einem Lumen positioniert war, wobei das Bildframe ein erstes Kriterium erfüllt, das einem anatomischen Merkmal zugeordnet ist, wobei die Identifizierung auf einem Vergleich mit einem Schwellenwert einer automatischen Messung des anatomischen Merkmals in der Vielzahl von Bildframes basiert; und
Durchführen mindestens eines des Folgenden:
- Identifizieren eines Bildframes der Vielzahl von Bildframes, das ein zweites Kriterium erfüllt, das dem anatomischen Merkmal zugeordnet ist, als ein proximales Kandidatenreferenzframe (506), das ein nächstgelegenes Gewebe des Lumens proximal zu dem Zielframe darstellt;
- wenn das proximale Kandidatenreferenzframe auf einem Seitenast liegt, distales Suchen, um das nächstgelegene Bildframe zu finden, das den Seitenast nicht beinhaltet, und Zuweisen des nächstgelegenen Bildframes als das proximale Referenzframe;
- wenn das proximale Kandidatenreferenzframe nicht auf einem Seitenast liegt, Zuweisen des proximalen Kandidatenreferenzframes als das proximale Referenzframe
und
- Identifizieren eines Bildframes der Vielzahl von Bildframes, das ein drittes Kriterium erfüllt, das dem anatomischen Merkmal zugeordnet ist, als ein distales Kandidatenreferenzframe (504), das ein nächstgelegenes Gewebe des Lumens distal zu dem Zielframe darstellt;
- wenn das distale Kandidatenreferenzframe auf einem Seitenast liegt, proximales Suchen, um das nächstgelegene Bildframe zu finden, das den Seitenast nicht beinhaltet, und Zuweisen des nächstgelegenen Bildframes als das distale Referenzframe; und
- wenn das distale Kandidatenreferenzframe keinen Seitenast umfasst, Zuweisen des distalen Kandidatenreferenzframes als das distale Referenzframe; und
Ausgeben, an eine Anzeige in Kommunikation mit der Prozessorschaltung, einer Einzelbildschirmanzeige, die Folgendes beinhaltet:
das Zielframe und jegliches zugewiesenes proximales Referenzframe und jegliches zugewiesenes distales Referenzframe; und
eine grafische Darstellung des Lumens (420), die eine jeweilige Position des Zielframes und jeglichen zugewiesenen proximalen Referenzframes und jeglichen zugewiesenen distalen Referenzframes zeigt.

9. Verfahren nach Anspruch 8, das unter Verwendung der Prozessorschaltung weiter Folgendes umfasst:
Durchführen mindestens eines des Folgenden:
- vor dem Zuweisen des nächstgelegenen Bildframes als das proximale Referenzframe oder das distale Referenzframe, Identifizieren, ob das nächstgelegene Bild ein weiteres Kriterium erfüllt, das dem anatomischen Merkmal zugeordnet ist; und
- wenn das nächstgelegene Bildframe das Kriterium erfüllt, Zuweisen des nächstgelegenen Referenzframes als das proximale Referenzframe oder das distale Referenzframe.

10. Verfahren nach Anspruch 8 oder 9, wobei das anatomische Merkmal aus mindestens einem Merkmal besteht, das aus der Gruppe ausgewählt wird, die aus einer Plaque-Belastung und einer Querschnittsfläche des Lumens besteht.

11. Verfahren nach einem der Ansprüche 8 bis 10, wobei das Verfahren weiter Folgendes umfasst:
Erzeugen, unter Verwendung der Prozessorschaltung in Kommunikation mit der intraluminalen Bildgebungsvorrichtung, der Vielzahl von Bildframes unter Verwendung der Bildgebungsdaten;
automatisches Messen, unter Verwendung der Prozessorschaltung, des anatomischen Merkmals in der Vielzahl von Bildframes.

12. Verfahren nach einem der Ansprüche 8 bis 11, wobei die automatische Messung als Reaktion darauf erfolgt, dass die Vielzahl von Bildframes erzeugt wird.

13. Verfahren nach einem der Ansprüche 8 bis 12, wobei die Einzelbildschirmanzeige einem Benutzer angezeigt wird, der die Anzeige verwendet.

14. Computerprogrammprodukt, das Anweisungen umfasst, die, wenn sie von einem Computersystem oder einer Computereinrichtung ausgeführt werden, das Computersystem oder die Computereinrichtung dazu veranlassen, ein beliebiges der Verfahren nach den Ansprüchen 8 bis 13 durchzuführen.

## Revendications

1. Système d'identification automatique de trame, comprenant un circuit de traitement (106) configuré pour :
identifier, comme trame cible (508) représentative d'une région d'intérêt, une trame d'image d'une pluralité de trames d'image générées à l'aide de données d'imagerie obtenues par un dispositif d'imagerie intraluminale (102) lorsqu'il est positionné dans une lumière, dans lequel la trame d'image satisfait à un premier critère associé à une caractéristique anatomique, dans lequel l'identification est basée sur une comparaison avec un seuil d'une mesure automatique de la caractéristique anatomique dans la pluralité de trames d'image ; et
effectuer au moins l'une parmi :
- l'identification, comme trame de référence proximale candidate (506) représentative d'un tissu le plus proche de la lumière à proximité de la trame cible, d'une trame d'image qui satisfait à un deuxième critère associé à la caractéristique anatomique ;
- si la trame de référence proximale candidate se trouve sur une branche latérale, la recherche distale pour trouver la trame d'image la plus proche qui n'inclut pas la branche latérale et la désignation de la trame d'image la plus proche comme trame de référence proximale ;
- si la trame de référence proximale candidate ne se trouve pas sur une branche latérale, la désignation de la trame de référence proximale candidate comme trame de référence proximale,
et
- l'identification, comme trame de référence distale candidate (504) représentative d'un tissu le plus proche de la lumière distale par rapport à la trame cible, d'une trame d'image qui satisfait à un troisième critère associé à la caractéristique anatomique ;
- si la trame de référence distale candidate se trouve sur une branche latérale, la recherche proximale pour trouver la trame d'image la plus proche qui n'inclut pas la branche latérale et la désignation de la trame d'image la plus proche comme trame de référence distale ; et
- si la trame de référence distale candidate ne comprend pas de branche latérale, la désignation de la trame de référence distale candidate comme trame de référence distale ; et
la production en sortie, vers un dispositif d'affichage en communication avec le circuit de traitement, d'un affichage sur écran unique incluant :
- la trame cible, et toute trame de référence proximale désignée et toute trame de référence distale désignée ; et
- une représentation graphique (420) de la lumière montrant une position respective de la trame cible et de toute trame de référence proximale désignée et de toute trame de référence distale désignée.

2. Système d'identification automatique de trame selon la revendication 1, dans lequel le circuit de traitement est en outre configuré pour :
effectuer au moins l'une parmi :
- avant la désignation de la trame d'image la plus proche comme trame de référence proximale ou comme trame de référence distale, l'identification pour établir si la trame d'image la plus proche satisfait à un critère supplémentaire associé à la caractéristique anatomique ; et
- si la trame d'image la plus proche satisfait au critère, la désignation de la trame de référence la plus proche comme trame de référence proximale ou trame de référence distale.

3. Système d'identification automatique de trame selon la revendication 1 ou 2, dans lequel la caractéristique anatomique consiste en au moins l'une choisie dans le groupe consistant en : une progression de plaque et une zone transversale de la lumière.

4. Système d'identification automatique de trame selon l'une quelconque des revendications 1 à 3, dans lequel le
circuit de traitement est en outre configuré pour :
générer la pluralité de trames d'image à l'aide des données d'imagerie ;
mesurer automatiquement la caractéristique anatomique dans la pluralité de trames d'image ;
communiquer avec un dispositif d'imagerie intraluminale configuré pour obtenir des données d'imagerie associées à une lumière d'un patient lorsqu'il est positionné dans la lumière.

5. Système d'identification automatique de trame selon l'une quelconque des revendications précédentes, dans lequel la mesure automatique est effectuée en réponse à la génération de la pluralité de trames d'image.

6. Système d'identification automatique de trame selon la revendication 1, comprenant le dispositif d'affichage en communication avec le circuit de traitement.

7. Système d'imagerie intraluminale comprenant :
un système d'identification automatique de trame selon l'une quelconque des revendications 1 à 6 ; et
le dispositif d'imagerie intraluminale.

8. Procédé d'identification automatique de trame, comprenant, à l'aide d'un circuit de traitement (106) :
l'identification, comme trame cible (508) représentative d'une région d'intérêt, d'une trame d'image d'une pluralité de trames d'image générées à l'aide de données d'imagerie obtenues par un dispositif d'imagerie intraluminale (102)
lorsqu'il est positionné dans une lumière, dans lequel la trame d'image satisfait à un premier critère associé à une caractéristique anatomique, dans lequel l'identification est basée sur une comparaison avec un seuil d'une mesure automatique de la caractéristique anatomique dans la pluralité de trames d'image ; et
l'exécution d'au moins l'une parmi :
- l'identification, comme trame de référence proximale candidate (506) représentative d'un tissu le plus proche de la lumière à proximité de la trame cible, d'une trame d'image de la pluralité de trames d'image qui satisfait à un deuxième critère associé à la caractéristique anatomique ;
- si la trame de référence proximale candidate se trouve sur une branche latérale, la recherche distale pour trouver la trame d'image la plus proche qui n'inclut pas la branche latérale et la désignation de la trame d'image la plus proche comme trame de référence proximale ;
- si la trame de référence proximale candidate ne se trouve pas sur une branche latérale, la désignation de la trame de référence proximale candidate comme trame de référence proximale,
et
- l'identification, comme trame de référence distale candidate (504) représentative d'un tissu le plus proche de la lumière distale par rapport à la trame cible, d'une trame d'image de la pluralité de trames d'image qui satisfait à un troisième critère associé à la caractéristique anatomique ;
- si la trame de référence distale candidate se trouve sur une branche latérale, la recherche proximale pour trouver la trame d'image la plus proche qui n'inclut pas la branche latérale et la désignation de la trame d'image la plus proche comme trame de référence distale ; et
- si la trame de référence distale candidate ne comprend pas de branche latérale, la désignation de la trame de référence distale candidate comme trame de référence distale ; et
la production en sortie, vers un dispositif d'affichage en communication avec le circuit de traitement, d'un affichage sur écran unique incluant :
la trame cible, et toute trame de référence proximale désignée et toute trame de référence distale désignée ; et
une représentation graphique de la lumière (420) montrant une position respective de la trame cible et de toute trame de référence proximale désignée et de toute trame de référence distale désignée.

9. Procédé selon la revendication 8, comprenant en outre, à l'aide du circuit de traitement :
l'exécution d'au moins l'une parmi :
- avant la désignation de la trame d'image la plus proche comme trame de référence proximale ou comme trame de référence distale, l'identification pour établir si la trame d'image la plus proche satisfait à un critère supplémentaire associé à la caractéristique anatomique ; et
- si la trame d'image la plus proche satisfait au critère, la désignation de la trame de référence la plus proche comme trame de référence proximale ou trame de référence distale.

10. Procédé selon la revendication 8 ou 9, dans lequel la caractéristique anatomique consiste en au moins l'une choisie dans le groupe consistant en : une progression de plaque et une zone transversale de la lumière.

11. Procédé selon l'une quelconque des revendications 8 à 10, dans lequel le procédé comprend en outre :
la génération, à l'aide du circuit de traitement en communication avec le dispositif d'imagerie intraluminale, de la pluralité de trames d'image à l'aide des données d'imagerie ;
la mesure automatique, à l'aide du circuit de traitement, de la caractéristique anatomique dans la pluralité de trames d'image.

12. Procédé selon l'une quelconque des revendications 8 à 11, dans lequel la mesure automatique est effectuée en réponse à la génération de la pluralité de trames d'image.

13. Procédé selon l'une quelconque des revendications 8 à 12, dans lequel l'affichage sur écran unique est affiché à l'attention d'un utilisateur à l'aide du dispositif d'affichage.

14. Programme informatique comprenant des instructions qui, lorsqu'elles sont exécutées par un système ou un appareil informatique, amènent le système ou l'appareil informatique à réaliser l'un quelconque des procédés selon les revendications 8 à 13.
